# EUROPEAN PATENT APPLICATION

(11) **EP 2 433 671 A1**
(43) Date of publication of application: **28.03.2012**
(21) Application number: 11180882.0
(22) Date of filing: 12.09.2011
(51) Int. Cl.: A61M 25/09

(54) **Guide wire for endoscope**

(30) Priority: 27.09.2010 JP 2010215470; 26.05.2011 JP 2011118318
(71) Applicant: Terumo Kabushiki Kaisha, Tokyo (JP)
(72) Inventor: Suzuki, Kenta, Sizuoka, Shizuoka (JP); Kobayashi, Junichi, Sizuoka, Shizuoka (JP); Kinoshita, Yasushi, Kanagawa, Shizuoka (JP)
(74) Representative: Dossmann, Gérard

(57) **Abstract**

A guide wire includes a wire having an elongated body section and a distal portion decreasing in outer diameter from the body section. The guide wire may have a resin coating portion coating the body section and the distal portion and having a smooth outer surface; and a visually discernible mark provided at the resin coating portion. The body section may have a flexible portion which constitutes a part of the body section and lower in flexural rigidity than other portions of the body section different from the part, and the visually discernible mark is provided at a position of the resin coating portion at which the flexible portion is coated with the resin coating portion

## Description

This application claims priority under 35 U.S.C. § 119 to Japanese Patent Application No. 2010-215470 filed on September 27, 2010, Japanese Patent Application No. 2010-218701 filed on September 29, 2010 and Japanese Patent Application No. 2011-118318 filed on May 26, 2011, the entire content of all three of which is incorporated herein by reference.

The invention described here relates to a guide wire for use with an endoscope which can be used for trans-endoscopically guiding a treatment instrument used in endoscopy or endoscopic surgery, particularly treatment of a pancreatobiliary duct system.

At present, a variety of treatments of the pancreatobiliary duct system using an endoscope are performed, such as imaging of the pancreatic duct or bile duct, removal of gallstone, securing of an opening of the duodenal papilla, etc. In the endoscopy or endoscopic surgery of the pancreatobiliary duct system, the endoscope is first inserted via the subject's mouth to the duodenal papilla constituting an entrance to the pancreatobiliary duct, and a catheter is trans-endoscopically inserted into the pancreatobiliary duct via a treatment instrument insertion channel in the endoscope. Thereafter, a guide wire is inserted into the pancreatobiliary duct via the catheter, and a part of the guide wire projecting out from the distal end of the endoscope is set indwelling in the pancreatobiliary duct. After the guide wire is thus put indwelling, the catheter is pulled out of the endoscope, and a device, for example another catheter, stent, papillotomy knife, stone basket, is inserted into the pancreatobiliary duct via the endoscope while being guided by the guide wire. Then, trans-endoscopic insertion and withdrawing of the catheter through the function of the guide wire are repeated until the treatment is completed.

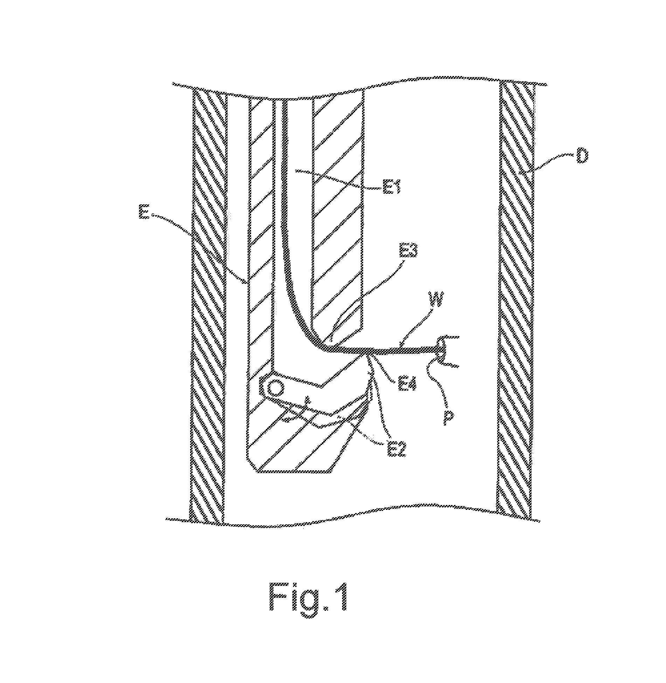

Normally, such catheter inserting and withdrawing operations require a few assistants in addition to the operator, and therefore involve difficulties as to cost, time, and sanitation. In view of this, an endoscope for assisting the catheter inserting and withdrawing operations is often used, as described for example in U.S. Application Publication No. 2002/0087100A1. This endoscope has at its distal end a mechanism by which a guide wire can be fixed, and the catheter replacing work is facilitated by fixation of the guide wire. To be more specific, as shown in FIG. 1, a claw-shaped member (treatment instrument elevator base) E2 for bending a guide wire W so as to veer sideways relative to the direction along the treatment instrument insertion channel E1 is turnably provided at the distal end of the treatment instrument insertion channel E1, and the guide wire W is held at two positions by the distal end E4 of the claw-shaped member E2 and a part E3 in the vicinity of the opening of the treatment instrument insertion channel E1. Here, the guide wire W is held in the state of being pressed against the distal end E4 of the claw-shaped member E2 and the part E3 in the vicinity of the opening, by its own reaction force with which it tends to return from the bent state to its original straight state.

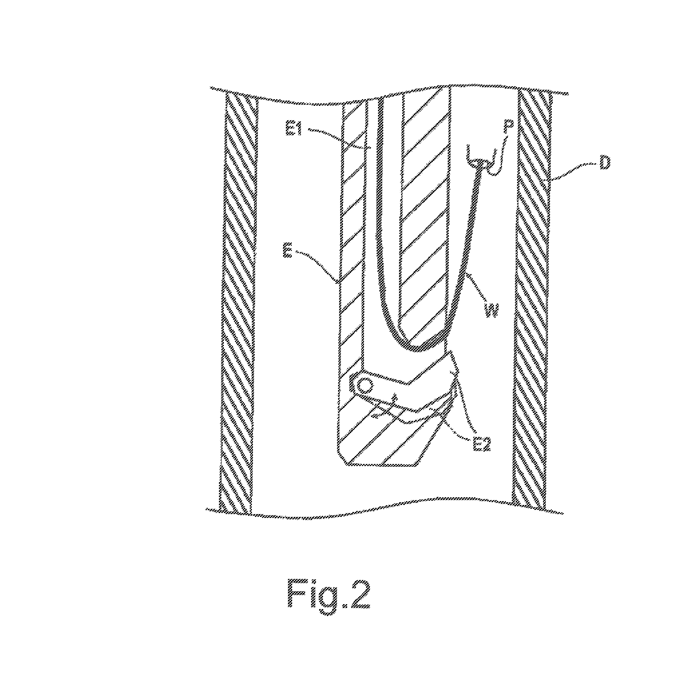

When the distal end of the endoscope E and the duodenal papilla P are remote from each other and the distal end of the endoscope E is held at such a position as to look up at the duodenal papilla P, as shown in FIG. 2, the guide wire W projecting from the distal end of the endoscope E is pulled in the direction opposite to the insertion direction of the endoscope E, resulting in that it is difficult for the guide wire W to make contact with the distal end E4 of the claw-shaped member E2. Depending on the position of the endoscope E, fixation of the guide wire W may thus be difficult to achieve, and the guide wire W may come out during the withdrawing of the catheter.

A guide wire disclosed here includes a wire having a body section and a distal portion, with the distal portion of the wire being distal of the body section and possessing a distal-most end, and with the distal portion decreasing in outer diameter toward the distal-most end of the distal portion. A resin coating portion covers at least a part of an axial extent of the body section and covers the distal portion. The resin coating portion forms an exposed outer surface of the guide wire, which exposed outer surface is a smooth outer surface. A visually discernible mark exists at the resin coating portion. This visually discernable mark is visually distinguishable from areas of the resin coating portion on opposite axial sides of the visually discernible mark. The body section includes a flexible portion which is a part of the body section, wherein the flexible portion is lower in flexural rigidity than other portions of the body section positioned on both axial ends of the flexible portion. The visually discernible mark is provided at a position of the resin coating portion which overlies the flexible portion so that the visually discernible mark axially overlaps the flexible portion.
The guide wire is useful with an endoscope and is constructed in a way that facilitates fixing the guide wire onto the endoscope independently of the positional relationship between the duodenal papilla and the endoscope.

Another aspect of the disclosure here involves a medical instrument comprising: an endoscope with a treatment instrument insertion channel and a treatment instrument elevator base at a distal end portion of the treatment instrument insertion channel, with the treatment instrument insertion channel possessing a proximal opening and a distal opening; and a guide wire insertable into the treatment instrument insertion channel by way of the proximal opening so that the guide wire extends along the treatment instrument insertion channel and a distal end portion of the guide wire extends distally beyond the distal opening of the treatment instrument insertion channel to be inserted into a duodenal papilla, the guide wire possessing an exposed outer surface. The guide wire includes a wire comprised of an elongated body section and a distal portion located distal of the body section and possessing an outer diameter decreasing in a distal direction of the distal portion. The guide wire also comprises a resin coating portion covering at least a part of an axial extent of the body section and the distal portion, the resin coating possessing an outer surface forming the exposed outer surface of the guide wire, the outer surface of the resin coating being smooth. The guide wire has a flexible portion lower in flexural rigidity than other portions of the guide wire, with the flexible portion being disposed along a length of the guide wire such that with the guide wire extending along the treatment instrument insertion channel and the distal end portion of the guide wire positioned in the duodenal papilla the flexible portion is distal of the opening.

An additional aspect of the disclosure here involves a method of using an endoscope to position a device in a duodenal papilla. The method involves: inserting the endoscope into a duodenum, the endoscope including a treatment instrument insertion channel having a proximal opening at one and a distal opening at an opposite end; moving the endoscope in the duodenum to position the distal opening below the duodenal papilla; and inserting a guide wire into the treatment instrument insertion channel of the endoscope. The guide wire includes a flexible portion lower in flexural rigidity than portions of the guide wire on both opposite axial ands of the flexible portion. The guide wire also comprises a wire and a resin coating portion having a smooth outer surface forming the outer surface of the guide wire, with the wire including an elongated body section and a distal portion located distal of the body section and possessing an outer diameter decreasing in a distal direction of the distal portion the resin coating portion covering at least a part of an axial extent of the body section and the distal portion. The method also involves moving the guide wire along the treatment instrument insertion channel so that a distal end of the guide wire exits the treatment instrument insertion channel through the distal opening and is positioned in the duodenal papilla, the flexible portion of the guide wire being positioned between the distal opening of the treatment instrument insertion channel and the duodenal papilla; and moving the device along the guide wire and inserting the device into the duodenal papilla.

The endoscopic guide wire according to the present invention configured as above is liable to be bent at the flexible portion. In the case where the distal end of the endoscope is held at such a position as to look up at the duodenal papilla, therefore, the flexible portion is drawn out of the endoscope and disposed between the distal end of the endoscope and the duodenal papilla, which ensures that in the condition where the distal side relative to the flexible portion of the guide wire is inserted in the duodenal papilla, the guide wire can be bent on the proximal side opposite to the distal side so as to make contact with the holding position of the endoscope. Accordingly, the function of fixing the guide wire onto the endoscope is easily displayed independently of the positional relationship between the duodenal papilla and the distal end of the endoscope.
In addition, the endoscopic guide wire has the visually discernible mark at the position of the resin coating portion at which the flexible portion is coated with the resin coating portion. This permits the operator to check the position of the flexible portion at the time of drawing out the flexible portion from the distal end of the endoscope. Accordingly, the guide wire is excellent in operationality.
Since the resin coating portion has the smooth outer surface and the treatment instrument is smoothly guided along the endoscopic guide wire, treatment instrument inserting and pulling-out operations can be easily carried out.
Where the flexible portion has a necked shape reduced in diameter as compared with the other portions, the endoscopic guide wire can be easily bent at the flexible portion into an arbitrary direction. This ensures that the above-mentioned fixing function can be displayed while flexibly corresponding to various positional relationships between the duodenal papilla and the endoscope.
Where the flexible portion has a groove formed in a spiral shape in an outer peripheral surface of the body section, the flexible portion is insusceptible to curling or kinking, so that the endoscopic guide wire can be inserted and pulled out easily.
Where the flexible portion is provided at a position spaced proximally from the distal end of the resin coating portion by not less than 150 mm, a certain length for insertion into the duodenal papilla is secured on the distal side relative to the flexible portion. This ensures that the guide wire portion on the distal side relative to the flexible portion is less liable to come out after inserted in the duodenal papilla.
Where the visually discernible mark has a radiopaque property, the position of the flexible portion can be grasped under radioscopy.
Where the body section has a plurality of the flexible portions, and intervals between the flexible portions are each not less than the spacing between two holding positions provided in the endoscope so as to hold the body section, the function of the flexible portion can be displayed if only any one of the plurality of flexible portions is disposed between the distal end of the endoscope and the duodenal papilla. This promises easier operations, as compared with the case where only one flexible portion is provided and it is to be disposed appropriately.
Where the flexible portion is coated with the resin coating portion, and a proximal-side resin coating portion composed of a resin member different from the resin coating portion is provided on the proximal side of the resin coating portion, there is obtained an effect such that the properties of the outer surface can be varied.
According to the medical instrument of the present invention configured as above, the guide wire is easily bent at the flexible portion. This ensures that even in the case where the distal end of the endoscope is held at such a position as to look up at the duodenal papilla, the guide wire can be bent on the proximal side opposite to the distal side so as to make contact with the holding position of the endoscope, in a condition where the guide wire portion on the distal side relative to the flexible portion is inserted in the duodenal papilla. Consequently, the function of fixing the guide wire onto the endoscope can be easily displayed independently of the positional relationship between the duodenal papilla and the distal end of the endoscope.

FIG. 1 is an enlarged view showing a condition in which a known guide wire is held by a holding part of an endoscope in the case where the distal end of the endoscope is at a position facing the duodenal papilla;

FIG. 2 is an enlarged view showing the manner in which a known guide wire is located remote from the holding part of the endoscope in the case where the distal end of the endoscope is located at a position looking up at the duodenal papilla;

FIG. 3 is a side view of a guide wire according to a first embodiment disclosed here.

FIG. 4 is a cross-sectional view of the guide wire shown in FIG. 3 taken along the section line 4-4 in FIG. 3.

FIG. 5 is a side view of a medical instrument according to one embodiment disclosed here.

FIG. 6 is an enlarged view of a distal portion of the medical instrument shown in FIG. 5.

FIG. 7 is a cross-sectional view of the distal portion of the medical instrument shown in FIG. 6.

FIG. 8 is a cross-sectional view of the distal portion of the medical instrument showing the manner in which an endoscope is inserted into the duodenum to a position looking up at the duodenal papilla.

FIG. 9 is a cross-sectional view of the distal portion of the medical instrument showing the manner in which a catheter is inserted into an endoscope inserted in the duodenum.

FIG. 10 is a cross-sectional view of the distal portion of the medical instrument showing the manner in which the catheter is drawn out from the distal end of the endoscope and inserted in the duodenal papilla.

FIG. 11 is a cross-sectional view of the distal portion of the medical instrument showing the manner in which the guide wire is inserted into the duodenal papilla through the catheter.

FIG. 12 is a cross-sectional view of the distal portion of the medical instrument showing the manner in which the guide wire is held, with its flexible portion disposed between the distal end of the endoscope and the duodenal papilla.

FIG. 13 is a cross-sectional view of the distal portion of the medical instrument showing the manner in which the catheter is inserted into a treatment instrument insertion channel of the endoscope along the guide wire.

FIG. 14 is a cross-sectional view of the distal portion of the medical instrument showing the manner in which the catheter is inserted into the duodenal papilla along the guide wire.

FIG. 15 is a side view of a guide wire according to a second embodiment disclosed here.

FIG 16 is a side view of a guide wire according to a third embodiment.

FIG. 17 is a side view of a guide wire according to a fourth embodiment.

FIG. 18 is a side view of a guide wire according to a fifth embodiment.

FIG. 19 is a side view of a guide wire according to a sixth embodiment.

FIG. 20 is a side view of a guide wire according to the sixth embodiment.

Various embodiments of the guide wire disclosed here by way of example are described below with reference to the accompanying drawing figures. The description of the second to fourth embodiments primarily discuss differences in the guide wires relative to the guide wire of the first embodiment. Features of the guide wire in subsequent embodiments that are the same as in earlier embodiments are identified by common reference numerals, and a detailed description of those common features is not repeated in the description of the subsequent embodiments. Similarly, the description of the fifth embodiment will primarily discuss differences in the guide wire relative to the guide wire of the fourth embodiment, and features of the fifth embodiment of the guide wire that are the same as in the fourth embodiment are identified by common reference numerals, and a detailed description of those common features is not repeated. The operating methods for an endoscope and a guide wire according to the second to sixth embodiments are substantially the same as the first embodiment, and so the description of the operating methods for the second to sixth embodiments is not repeated. The size ratios or relative sizes in the drawings are exaggerated for convenience of illustration and ease in understanding and so it should be understood that they may be different from the actual ratios and relative sizes.

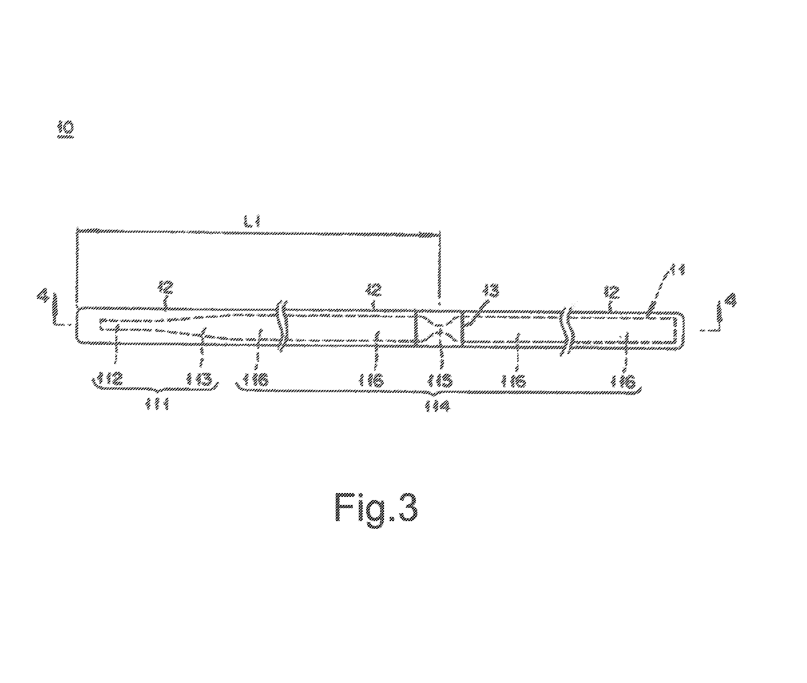

Referring to FIG. 3, a guide wire 10 according to the first embodiment is for trans-endoscopically guiding a catheter used in endoscopy or endoscopic surgery of the pancreatobiliary duct system, and includes an elongated flexible wire 11 and a resin coating portion 12 coating or covering the wire 11. The length of the guide wire 10 is 1,500 to 5,000 mm, for example.

The wire 11 has an elongate body section 114 and a distal portion 111 extending distally from the distal end of the body section 114 and having a smaller outer diameter that the outer diameter of the body section 114. The material forming the wire 11 is a metallic material such as superelastic alloys, for example nickel-titanium alloy, copper-zinc alloy, etc. or stainless steel, or a resin material having a comparatively high rigidity.

The distal portion 111 includes a taper portion 113 having a tapered shape and formed integrally in one piece as a unitary construction with the body section 114, and a distal reduced-diameter portion 112 extending axially in the distal direction from the taper portion 113 and formed integrally and in one piece as a unitary construction. Both the taper portion 113 and the distal reduced-diameter portion 112 have a circular cross-sectional shape throughout their entire lengths. Alternately the distal reduced-diameter portion 112 may have a tip with a plate-like cross-sectional shape. In this embodiment disclosed by way of example, the distal reduced-diameter portion 112 is circular in cross-sectional shape, but the guide wire is not limited to this configuration. The distal reduced-diameter portion 112 may be formed by press working so that it possesses a rectangular or barrel-shaped cross-section.

The body section 114 includes an intermediately located flexible portion 115 where the flexural rigidity of the body section 114 is locally lowered or reduced. The flexible portion 115 constitutes a part of the body section 114, and is lower in flexural rigidity than all other portions 116 of the body section 114. The flexible portion 115 and the other portions 116 have a circular or barrel-like cross-sectional shape, and the flexible portion 115 has a necked shape reduced in outer diameter as compared with all the other portions 116 of the body section 114. The flexible portion 115 is preferably configured so that it does not bend under its own weight when the guide wire 10 is held horizontally at several centimeters proximally when the place of holding is several centimeters on the proximal side of the proximal end of the flexible portion 115 of the guide wire. The flexible portion 115 thus maintains a straight shape when no outside load is applied to the guide wire.

The position of the flexible portion 115 in the guide wire 10 is preferably so set that the flexible portion is located on the distal side relative to a treatment instrument elevator base when the guide wire 10 is inserted and passed in a treatment instrument insertion channel of an endoscope E described later and the distal portion 111 of the guide wire 10 is inserted in a duodenal papilla P. The distance L1 from the distal end to the center of the flexible portion 115 in the longitudinal direction of the guide wire 10 is preferably 50 to 250 mm, more preferably 100 to 200 mm, further more preferably 150 mm.

The resin coating portion 12 coating or covers the wire 11 entirely, That is, in the illustrated embodiment, the resin coating portion 12 covers the entirety of the wire. Examples of the material forming the resin coating portion 12 include various thermoplastic resins and thermosetting resins such as polyvinyl chloride, polyolefins such as polyethylene, polypropylene, ethylene-propylene copolymer, ethylene-vinyl acetate copolymer, etc., polyesters such as polyethylene terephthalate, polybutylene terephthalate, etc., polyurethane, polyamides, polyimides, fluororesins such as polytetrafluoroethylene, polyvinylidene fluoride, etc., thermoplastic elastomers such as polyamide elastomers, polyester elastomers, etc., and various rubbers. Besides, the resin coating portion 12 contains a radiopaque material such as tungsten, barium sulfate, etc. This helps ensure that the position of the distal end of the guide wire 10 can be securely grasped under radioscopy.

A visually discernible mark 13 is formed at a position of the resin coating portion 12 at which the flexible portion 115 is coated with the resin coating portion 12. The visually discernible mark 13 at least partially axially overlaps the flexible portion 115 so that the visually discernible mark 13 overlies at least a part of the flexible portion 115. The visually discernable mark 13 is visually distinguishable from areas of the resin coating portion on opposite axial sides of the visually discernible mark. The axial extent of the visually discernible mark 13 is the same as or greater than the axial extent of the flexible portion 115. The visually discernible mark 13 is an annularly shaped mark extending around the entire circumference of the wire 11. With the mark 13 thus formed over the whole circumference of the wire 11, the mark 13 would not be lost sight of even where the guide wire 10 is rotated. Accordingly, the guide wire 10 exhibits relatively excellent operationality.

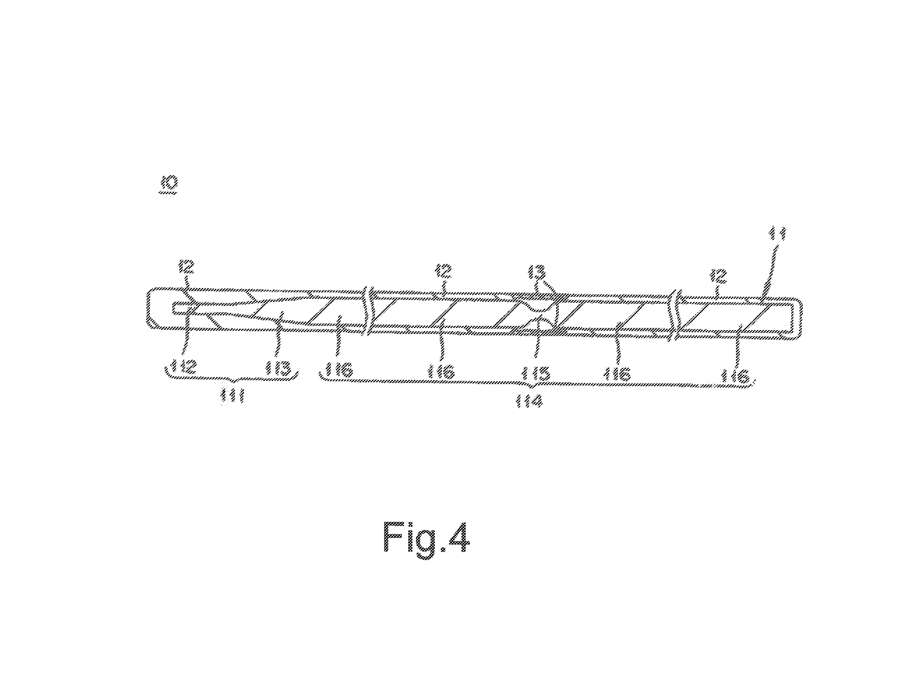

The mark 13 is formed, for example, by printing or by addition of a pigment so that the mark 13 is different in color from the other portions of the resin coating portion 12. The mark 13 may be formed by a method in which the resin coating portion 12 containing particulates of a carbon material is irradiated with a laser beam so that a part of the resin coating portion 12 changes color. As shown in FIG. 4, the outer surface of the resin coating portion 12 is smooth and rectilinear in the axial direction. That is, the outer surface of the guide wire 10 (i.e., the outer surface of the resin coating portion 12) is devoid of undulations or variations in outer dimensions (outer diameter) along the axial extent of the of the guide wire 10. In addition, the diameter of the guide wire 10 is substantially constant along its axial extent.

The description now turns to the guide wire 10 and medical instrument 1 having an endoscope E to be used with the guide wire 10.

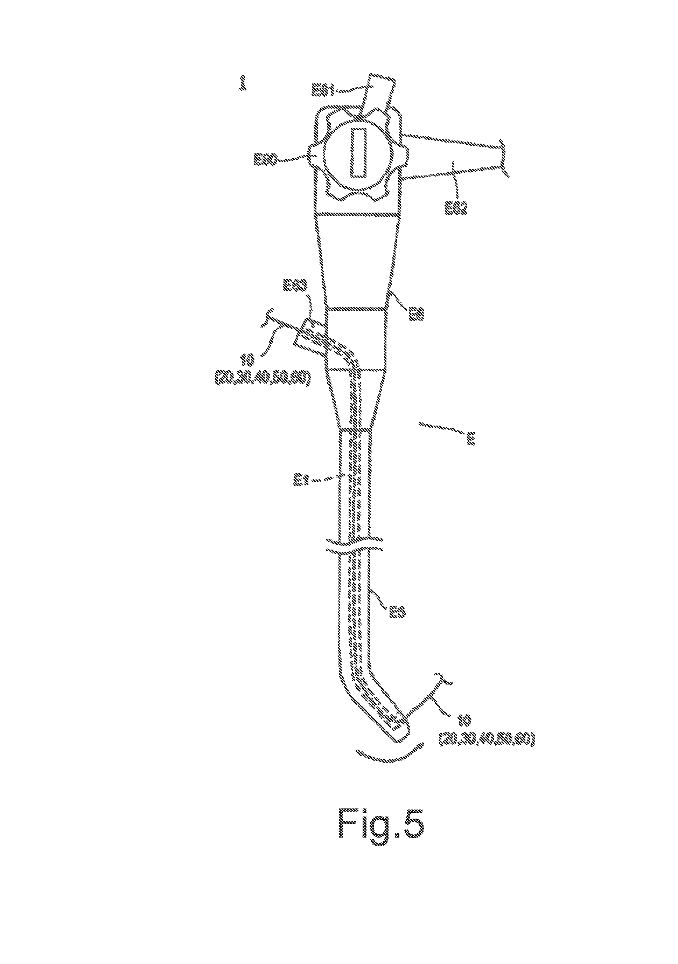

As shown in FIG 5, the medical instrument 1 includes a guide wire 10, and the endoscope E in which the guide wire 10 can be inserted and passed.

The endoscope E includes a flexible elongated insertion section E5 configured to be inserted into a living body, and an operating section E6 connected to the proximal end of the insertion section E5 and used for operating the insertion section E5.

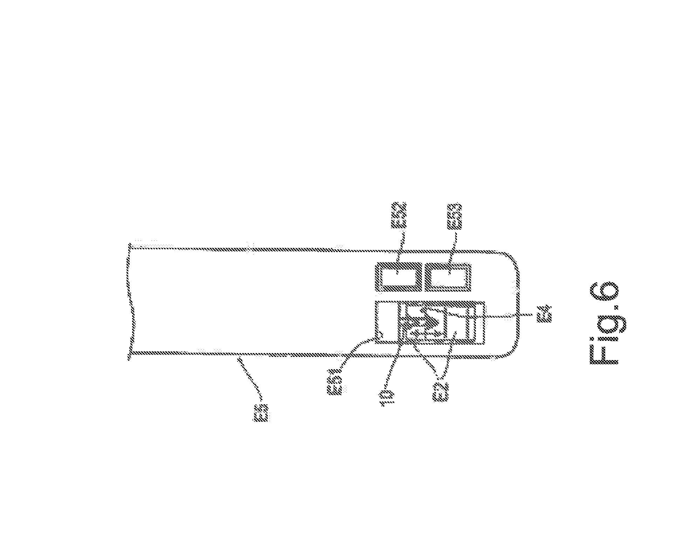

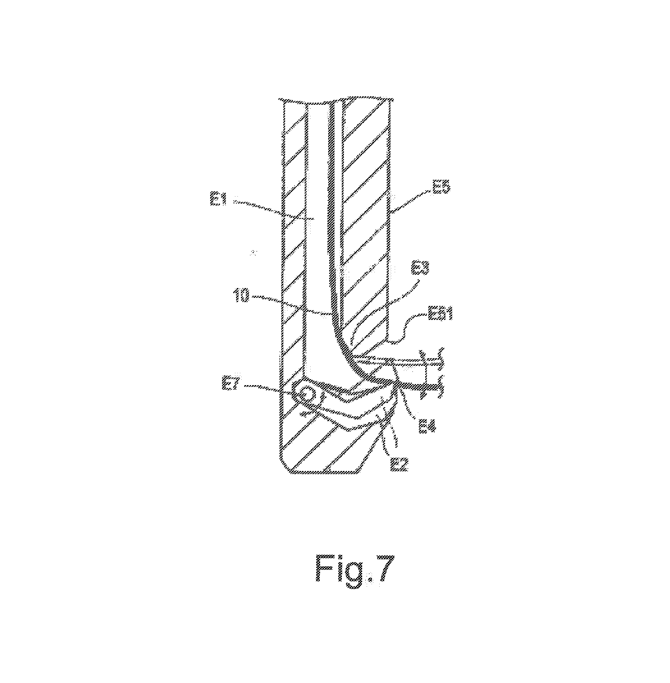

The insertion section E5 has a treatment instrument insertion channel E1 which extends along the insertion section E5 and in which the guide wire 10 can be inserted and along which the guide wire 10 can be moved. As shown in FIGS. 6 and 7, the distal end of the insertion section E5 has an opening E51 communicating with the treatment instrument insertion channel E1, and a treatment instrument elevator base E2 for regulating or varying the direction of the distal end of the guide wire 10 or catheters drawn out from the endoscope E and for holding the guide wire 10 or catheters. The distal end of the insertion section E5 includes an image pick-up section E52 for picking up an image of the inside of a living body, and an illumination section E53 juxtaposed to the image pick-up section E52 so as to illuminate the inside of the living body. The image pick-up section E52 has a CCD sensor, for example.

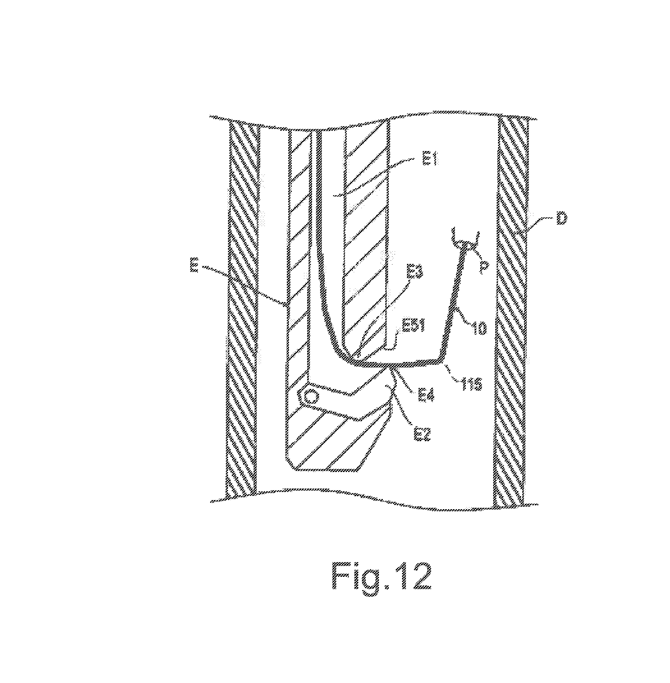

The opening E51 (the axis of the opening) is transverse to the direction of orientation of the insertion section E5. IN the illustrated embodiment, the opening E51 is oriented in a direction substantially orthogonal to the direction along the insertion section E5. The guide wire 10 passing through the treatment instrument insertion channel E1 is drawn out via the opening E51, and is inserted into the duodenal papilla. In this condition, the flexible portion 115 of the guide wire 10 is on the distal side relative to the treatment instrument elevator base E2 as described below in Fig.12.

The treatment instrument elevator base E2 is a claw-shaped member. The treatment instrument elevator base E2 is turnably supported on a shaft E7 at a distal portion of the insertion section E5. The treatment instrument elevator base E2, when it is turned or rotates about the shaft E7, bends the guide wire 10 to thereby change the extending direction of the distal end of the guide wire 10.

The guide wire 10 bent by the treatment instrument elevator base E2 contacts an inside corner portion E3 (holding position), at which the treatment instrument insertion channel E1 curves from the direction along the insertion section E5 toward the opening E51, and contacts a distal end E4 (holding position) of the treatment instrument elevator base E2. The guide wire 10 is held by being pressed against the one part E3 (comer portion) of the treatment instrument insertion channel E1 and the other part E4 (distal end) of the treatment instrument elevator base E2 by its own reaction force with which it tends to return from the bent state to its original straight state.

The image pick-up section E52 and the illumination section E53 are oriented in a direction substantially orthogonal to the direction along the insertion section E5. In other words, the endoscope E is of the side viewing type in which observation, or viewing, is made in a direction substantially orthogonal to the direction along the insertion section E5.

The operating section E6 of the endoscope E includes a knob E60 for bending the distal end of the insertion section E5, a lever E61 for operating the treatment instrument elevator base E2, and a treatment instrument insertion section E63 communicating with the treatment instrument insertion channel E1. The operating section E6 is provided with a universal cord E62 for sending signals from the image pick-up section E52 to a display device including a display and a video processor, etc. and for directing light from a light source to the illumination section E53.

By operating the knob E60, the distal end of the insertion section E5 is bent into a desired direction. The lever E61 is connected to the treatment instrument elevator base E2 through wires. With the lever E61 operated, the treatment instrument elevator base E2 is moved to rotate about the shaft E7.

A method of operating the guide wire 10 is as follows. In brief, the method of operating the guide wire 10 includes: an endoscope insertion step of inserting the endoscope into the duodenum until the distal end of the endoscope E reaches a position at which it is looking up at the duodenal papilla; a guide wire insertion step in which, after the endoscope insertion step, the guide wire 10 is inserted into the duodenal papilla through the endoscope E inserted in the duodenum so that the flexible portion 115 is located between the opening E51 and the duodenal papilla; and a device insertion step in which, after the guide wire insertion step, the device is moved ahead along the guide wire 10, in the condition where the guide wire 10 is held in position, whereby the device is inserted into the duodenal papilla.

The method of operating the guide wire 10 can also include a catheter insertion step in which a catheter for guiding the guide wire 10 to the duodenum papilla is inserted into the endoscope E, after the endoscope insertion step and before the guide wire insertion step. Each of the above-mentioned aspects of the method is described below in more detail.

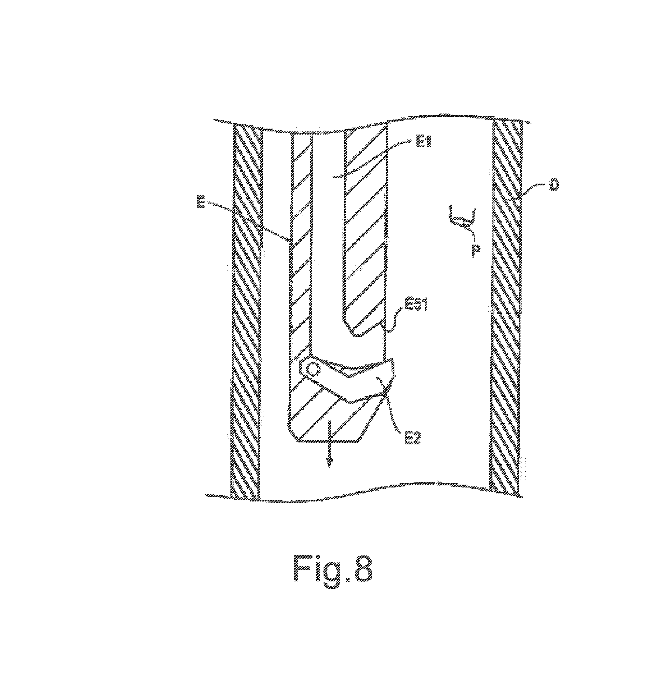

As shown in FIG. 8, in the endoscope insertion step, the operator inserts the endoscope E through the subject's mouth, and advances the endoscope E into the duodenum D while checking the position of the distal end of the endoscope E on the basis of an image obtained by the image pick-up section E52. The operator stops the insertion of the endoscope E when the distal end of the endoscope E in the duodenum D has reached a position past the duodenal papilla P, namely when the distal end of the endoscope E has reached such a position as to look up at the duodenal papilla P. In other words, the duodenal papilla P is positioned above the opening E51 as shown in FIG. 8.

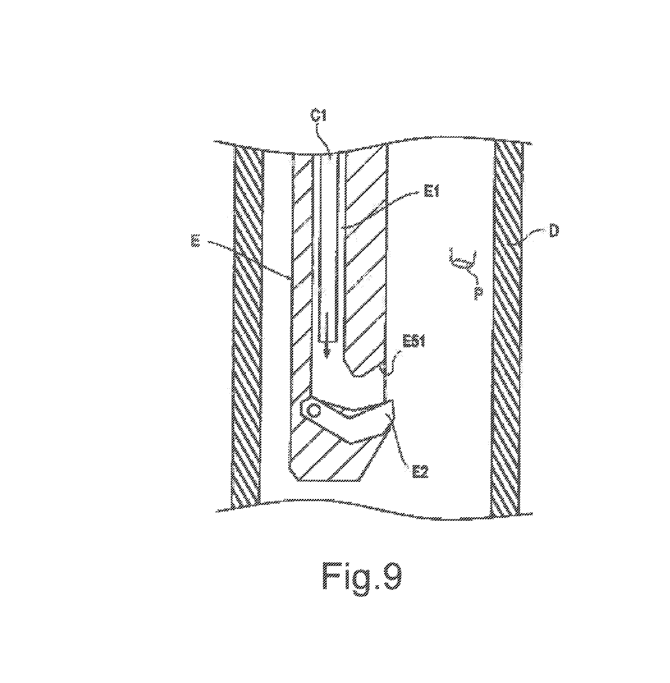

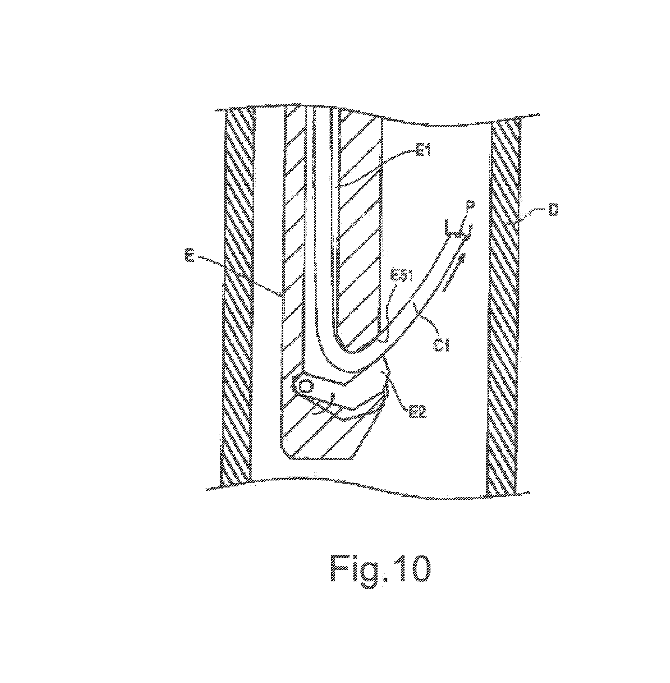

As shown in FIG 9, in the catheter insertion step following the endoscope insertion step, the operator inserts a flexible tubular catheter C1 into the treatment instrument insertion channel E1 via the treatment instrument insertion section E63 shown in FIG. 5. Then, as shown in FIG. 10, the operator moves the catheter so that the distal end of the catheter protrudes distally beyond the opening E51, and regulates the orientation of the catheter C1 to insert the catheter C1 into the duodenal papilla P. The operator operates the lever E61 so as to turn the treatment instrument elevator base E2, thereby regulating the orientation of the catheter C1.

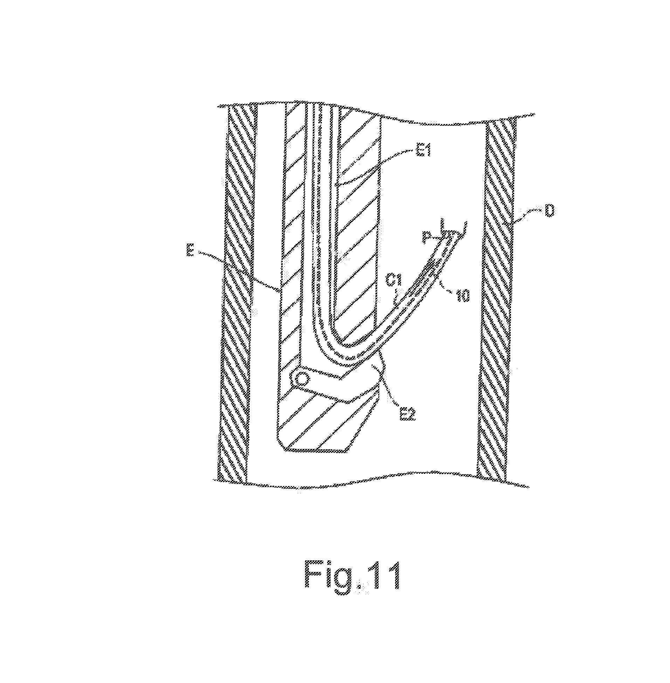

As shown in FIG. 11, in the guide wire insertion step following the catheter insertion step, the operator inserts the guide wire 10 into the duodenal papilla P and further into the bile duct or the pancreatic duct, via the lumen of the catheter C1. That is, the guide wire is inserted into the treatment instrument insertion channel E1 and is moved along the treatment instrument insertion channel so that the distal end of the guide wire exits the treatment instrument insertion channel through the distal opening E51 and is positioned in the duodenal papilla P.

After the guide wire 10 is thus inserted, the operator pulls out the catheter C1 while keeping the guide wire 10 in the inserted state. The operator regulates the position of the flexible portion 115 of the guide wire so that the flexible portion 115 is located at a position on the distal side of the opening E51 and on the proximal side of the duodenal papilla P, namely at a position between the opening E51 and the duodenal papilla P, as shown in FIG. 12. Because the guide wire 10 is provided with the mark 13, the operator can relatively easily check and regulate the position of the flexible portion 115 while viewing the image obtained by the image pick-up section E52. In the condition where the flexible portion 115 is located between the duodenal papilla P and the opening E51, the operator holds the guide wire 10 (i.e., the guide wire 10 is held) by the distal end E4 of the treatment instrument elevator base E2 and the corner portion E3.

The angle of the flexible portion 115 of the guide wire 10 may be changed according to the positional relationship between the opening E51 and the duodenal papilla P. With the positional relationship shown in Fig.1, the flexible portion 115 may not need to be bent in case the opening E51 is relatively close to the duodenal papilla P. On the other hand, as shown in Fig. 12, the flexible portion 115 may be bent towards the duodenal papilla P while holding the guide wire by the distal end E4 of the treatment instrument elevator base E2 when the opening E51 is relatively far from the duodenal papilla P. In Fig. 12, a curvature radius of the flexible portion 115 is smaller than a curvature radius of the guide wire 10 from the treatment instrument insertion channel E1 to the opening E51, Since the angle of the flexible portion 115 of the guide wire 10 may be readily changed because of the flexible portion 115, the operator may choose an intended endoscope position within the duodenum D.

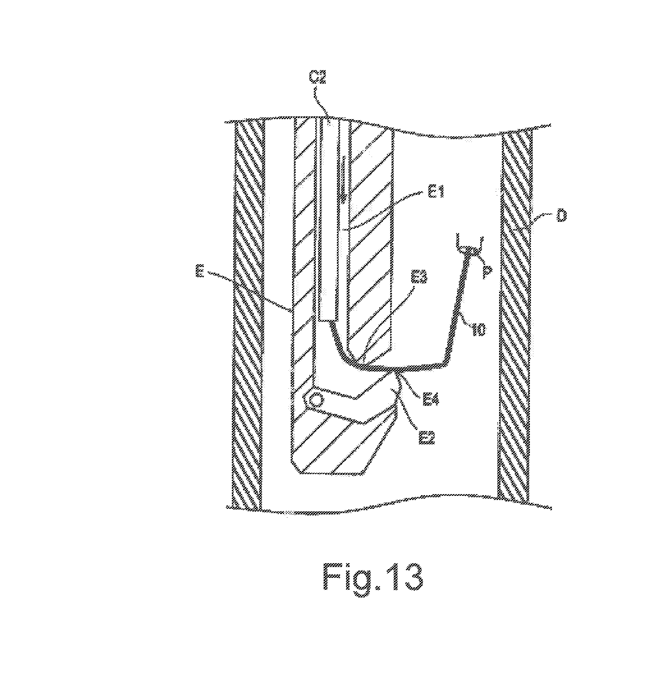

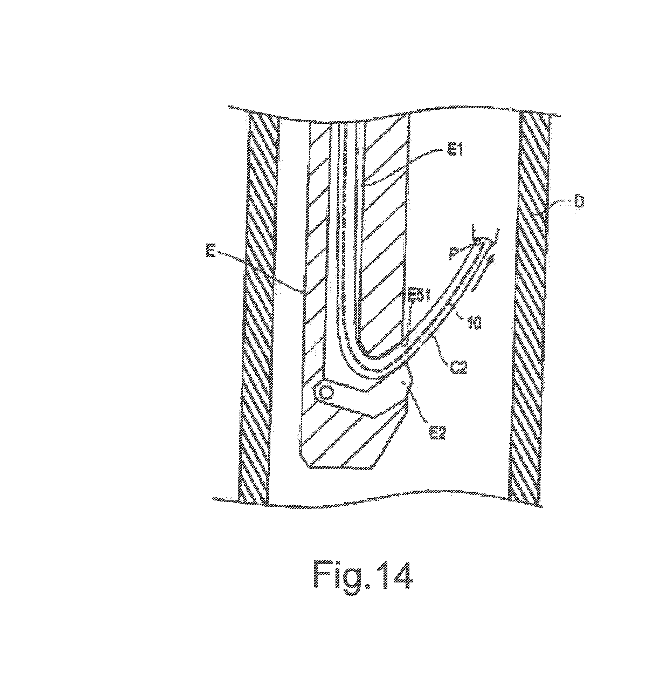

As shown in FIGS. 13 and 14, in the insertion step, the operator inserts a device required for the treatment such as diagnosis, surgery, etc., for example a catheter C2 equipped with a stent, a papillotomy knife, a basket for catching/removing gallstones, or the like, along the guide wire 10. The operator inserts the catheter C2 to a predetermined position in the bile duct or the pancreatic duct, and performs the desired treatment such as removal of gallstone. Since the guide wire 10 is held in the state in which is contacts the distal end E4 of the treatment instrument elevator base E2 and the corner portion E3, it can be fairly easily maintained in the inserted state in which the guide wire 10 is inserted in the duodenal papilla P even when the catheter C2 is inserted and pulled out.

The guide wire 10 can be bent at its flexible portion 115 and so even in the case where the distal end of the endoscope E is held at a position looking up at the duodenal papilla P, the arrangement of the flexible portion 115 between the distal end of the endoscope E and the duodenal papilla P as shown in FIG. 12 helps ensure that in the condition where the guide wire portion on the distal side of the flexible portion 115 is inserted in the duodenal papilla P, the guide wire portion on the proximal side opposite to the distal side can be bent to make contact with the corner portion E3 and the distal end E4 of the treatment instrument elevator base E2. Consequently, the fixing of the guide wire 10 onto the endoscope E can be fairly easily exhibited independently of the positional relationship between the distal end of the endoscope E and the duodenal papilla P.

Because the flexible portion 115 does not bend under its own weight as mentioned above, the guide wire 10 is not excessively bendable and thus does not exhibit less operationality. Therefore the guide wire 10 may be securely held by being pressed against the distal end E4 of the treatment instrument elevator base E2 by its own adequate reaction force.

Because the flexible portion is kept in a straight shape unless a bending load is applied, the guide wire 10 is able to achieve the aforementioned results while still exhibiting ordinary operationality.

In this embodiment, the wire 11 is covered with the resin coating portion 12 so that the guide wire 10 may be prevented from being caught on the treatment instrument elevator base E2. The guide wire inserting and pulling-out operations can be carried out smoothly, and the guide wire 10 is thus inhibited or prevented from being damaged.

Because the outer surface of the resin coating portion 12 is smooth and the catheter C2 is smoothly guided along the guide wire 10, the operations involving inserting and pulling-out the catheter C2 can be carried out relatively easily.

The mark 13 on the guide wire 10 allows the position of the flexible portion 115 to be relatively easily checked based on the function of the image pick-up section E52 provided at the distal end of the endoscope E, and quite excellent operationality is secured, during when the operator draws out the flexible portion 115 from the opening E51 at the distal end of the endoscope E and disposes the flexible portion 115 between the distal end of the endoscope E and the duodenal papilla P.

Because the resin coating portion 12 contains a radiopaque material composed of a powdery inorganic material such as tungsten, barium, sulfate, bismuth oxide, etc., a contrast in radioscopy is generated between the resin coating portion 12 and the mark 13 which does not contain any radiopaque material or contains a lesser amount of radiopaque material than that of the resin coating portion 12. This helps ensure that the distance from the distal end of the guide wire 10 can be measured and that the guide wire 10 can be accurately disposed in a target site even in the case where the mark 13 comes out of the field of vision of the image pick-up section E52.

The flexible portion 115 is necked shaped, having a reduced outer diameter as compared with the other portions 116 of the guide wire, and so the guide wire 10 can be relatively easily bent at the flexible portion 115 in a desired direction. The reduced outer diameter of the flexible portion 115 extends around the entire circumferential extent of the flexible portion 115 so that the flexible portion can be bent in any direction. The above-described fixation of the guide wire can thus be more successfully exhibited while flexibly corresponding to various positional relationships between the duodenal papilla P and the endoscope E, as compared with a case in which the guide wire can only be bent in a fixed direction.

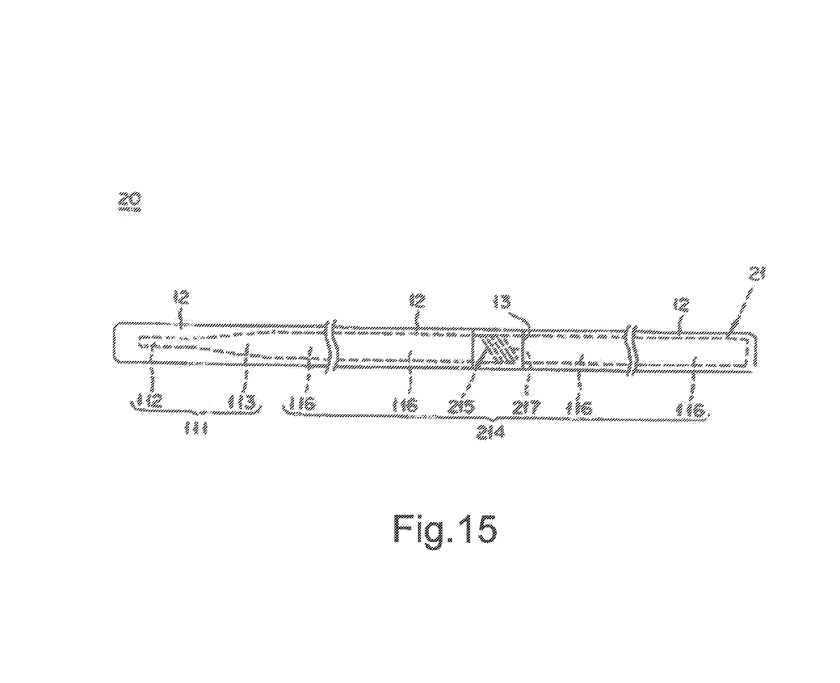

A second embodiment of the guide wire shown in FIG. 15 is roughly the same as the first embodiment, but is different from the first embodiment in the configuration of the flexible portion of the wire. The endoscope E and the operating method in this second embodiment are the same as in the first embodiment, and so the description of the endoscope and the operating method will not be repeated.

As shown in FIG. 15, a flexible portion 215 in the second embodiment is not neck-shaped (reduced in outer diameter), but is equal in outer diameter to portions 116 of the body section 214 other than the flexible portion 215. The flexible portion 215 has a spiral-shaped groove 217 in the outer peripheral surface of the body section 214.

Therefore, the guide wire 20 in the second embodiment is insusceptible to plastically deforming at the flexible portion 215. Thus, in addition to the benefits associated with the first embodiment of the guide wire discussed above, the second embodiment of the guide wire 20 can be inserted and pulled out relatively easily.

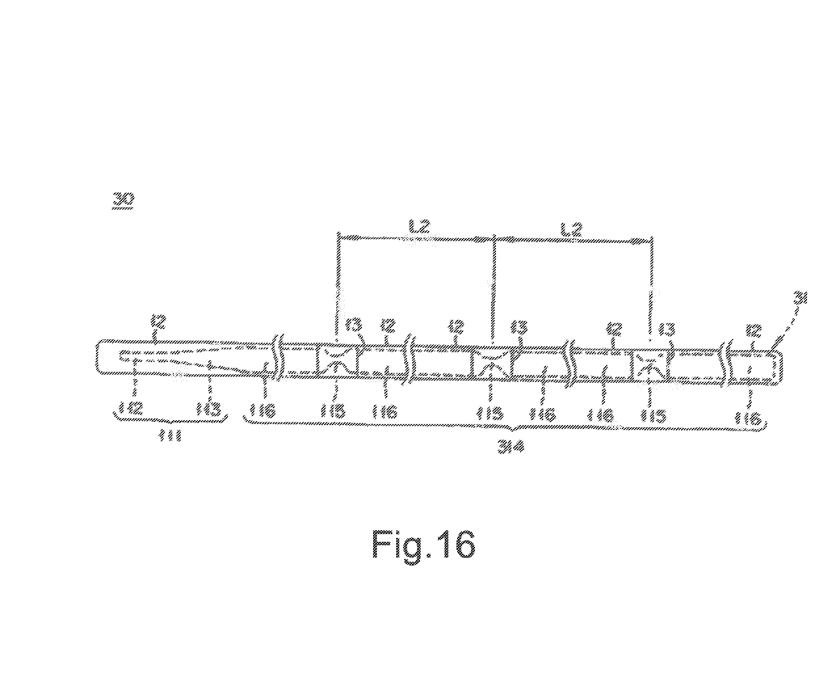

A third embodiment of the guide wire shown in FIG. 16 is roughly the same as the first embodiment, but is different from the first embodiment in that the wire has a plurality of flexible portions and marks. The endoscope E and the operating method in this third embodiment are the same as in the first embodiment, and so the description of the endoscope and the operating method is not repeated.

As shown in FIG. 16, the guide wire 30 in the third embodiment includes a body section 314 of a wire 31 having a plurality of flexible portions 115 spaced axially apart from one another along the longitudinal extent of the guide wire. The axial interval or distance L2 between axially adjacent flexible portions 115 is not less than the spacing or distance between the corner portion E3 and the distal end E4 of the treatment instrument elevator base E2.

In the guide wire 30 in the third embodiment, therefore, the function of the flexible portion 115 is displayed if any one of the plurality of flexible portions 115 is located between the distal end of the endoscope E and the duodenal papilla P. Accordingly, the operation of the guide wire is easier, as compared with the case where only one flexible portion is provided. In addition, because the third embodiment is the same as the first embodiment except for the plurality of flexible portions 115 and the associated plural marks 13, the guide wire 30 in the third embodiment exhibits benefits similar to those associated with the first embodiment described above.

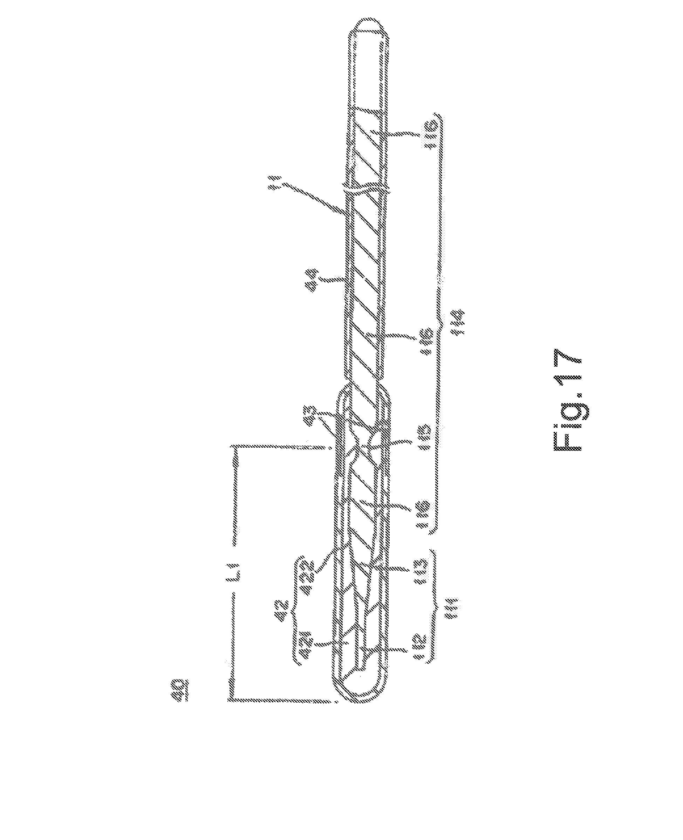

Referring to FIG. 17, a fourth embodiment of the guide wire 40 includes a flexible wire 11 similar to that in the first embodiment, but differing from the first embodiment in that the wire 11 is coated with a resin coating portion 42 (first resin coating portion) and a proximal-side resin coating portion 44 (second resin coating portion) which are different from each other. The endoscope E and the operating method in the fourth embodiment are the same as in the first embodiment, and so a description of the endoscope and the operating method is not repeated.

The resin coating portion 42 includes a first coating material 421 which coats or covers the wire portion ranging from a distal portion 111 to the flexible portion 115 (inclusive of the flexible section 115) and a part of a body section 116 on the proximal side of the flexible portion 115, and a second coating material 422 which entirely coats or covers the first coating material 421.

The outer surface of the resin coating portion 42 is smooth, and the resin coating portion 42 is provided with a visually discernible mark 43 at a position at which the flexible portion 115 is coated with the resin coating portion 42. The mark 43 is configured in the same manner as the mark 13 in the first embodiment. The mark 43 is visually distinguishable from the immediately adjoining portions of the outer surface of the resin coating portion 42.

Examples of the material which can be used as the first coating material 421 include polyolefins such as polyethylene, polypropylene, etc., polyvinyl chloride, polyesters (PET, PBT, etc.), polyamides, polyimides, polyurethane, polystyrene, silicone resins, thermoplastic elastomers such as polyurethane elastomer, polyester elastomers, polyamide elastomers, etc., various rubber materials such as latex rubber, silicone rubbers, etc. and composite materials obtained by combining two or more of these materials, among which preferred is polyurethane.

The second coating material 422 is formed from a hydrophilic material. Examples of the hydrophilic material include cellulose polymeric materials, polyethylene oxide polymeric materials, maleic anhydride polymeric materials (e.g., maleic anhydride copolymers such as methyl vinyl ether-maleic anhydride copolymer), acrylamide polymeric materials (e.g., polyacrylamide, polyglycidyl methacrylate-dimethylacrylamide (PGMA-DMAA) block copolymer), water-soluble nylon, polyvinyl alcohol, and polyvinyl pyrrolidone.

The second coating material 422 contains a radiopaque material such as tungsten, barium sulfate, etc. Therefore, the position of the distal end of the guide wire can be securely grasped or identified under radioscopy, and a contrast under radioscopy is generated between the distal end and the mark 43 which does not contain any radiopaque material.

In this embodiment, the flexible portion 115 is provided at a position spaced proximally from the distal-most end of the resin coating portion 42 by not less than 150 mm (i.e., L1 is not less than 150 mm). The distance L1 from the distal end of the resin coating portion 42 to the flexible portion 115 is preferably 150 to 300 mm, more preferably 150 to 200 mm. Correspondingly to this, the length of the resin coating portion 42 along the axial direction is preferably 250 to 500 mm, and more preferably 250 to 350 mm. Because the flexible portion 115 is provided at a position spaced proximally from the distal end of the resin coating portion 42 by at least 150 mm, a certain length for insertion into the duodenal papilla can be secured on the distal side of the flexible portion 115, favorably resulting in the guide wire portion on the distal side of the flexible portion 115 would not easily come out after insertion in the duodenal papilla.

The proximal-side resin coating portion 44 is provided on the proximal side of, and as a separate body from, the resin coating portion 42. The proximal-side resin coating portion 44 coats or covers the circumference of the body section 114 entirely. The proximal-side resin coating portion 44 is formed from a resin member or resin material different from the resin coating portion 42. Examples of the material forming the proximal-side resin coating portion 44 include fluororesin materials such as polytetrafluoroethylene (PTFE), fluorinated ethylene propylene (FEP), etc., among which preferred is polytetrafluoroethylene (PTFE).

Thus, the guide wire 40 in this embodiment is configured in roughly the same manner as in the first embodiment, but with the resin coating portion 42 and the proximal-side resin coating portion 44. Therefore, in addition to realizing benefits such as those discussed above with respect to the first embodiment, the properties of the guide wire outer surface can be varied. In this embodiment, the resin coating portion 42 has the second coating material 422 containing the hydrophilic material, so that lubricity of the outer surface is exhibited in a wetted state, and frictional resistance on the outer surface is reduced.

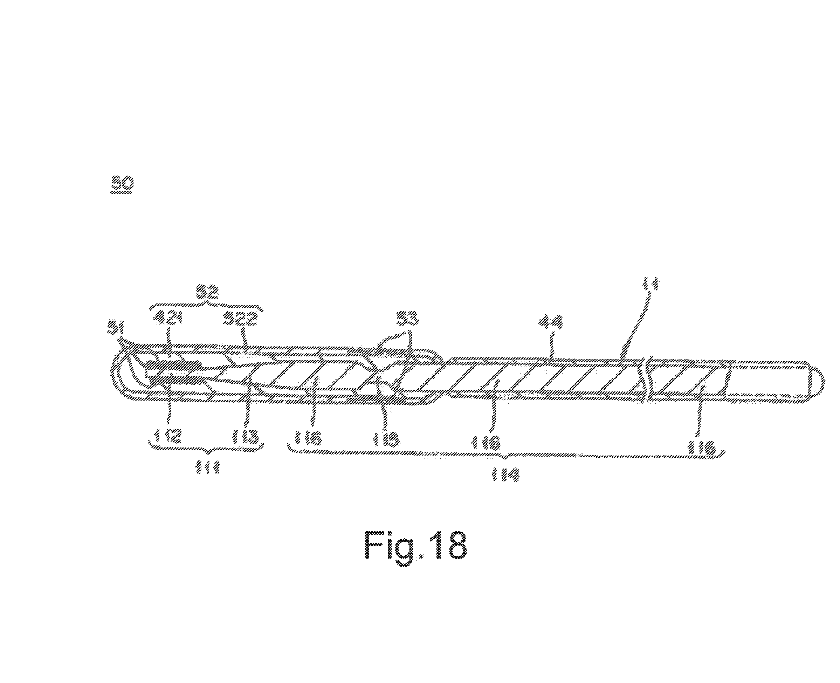

FIG. 18 illustrates a guide wire 50 according to a fifth embodiment that is roughly the same as the guide wire 40 in the fourth embodiment, but differs from the fourth embodiment in that it has a radiopaque coil 51. The guide wire 50 also differs from the fourth embodiment in that a visually discernible mark 53 is radiopaque, whereas a resin coating portion 52 provided with the mark 53 is not radiopaque. In all other respects, the fifth embodiment of the guide wire 50 is the same as the fourth embodiment. The endoscope E and the operating method in the fifth embodiment are the same as in the first embodiment, and so a description of those aspects is not repeated.

The coil 51 is formed, for example, from a noble metal such as gold, platinum, tungsten, etc. or an alloy containing such a noble metal (e.g., platinum-iridium alloy) or the like. The coil 51 is wound in solid coiling around a distal small-diameter portion 112 (i.e., the axially adjacent windings of the coil 51 contact one another), and is entirely coated with the resin coating portion 52. The coil 51 may also be disposed around the taper portion 113. That is, the coil 51 may be wound around the distal small-diameter portion 112, or the taper portion 113, or both the taper portion 113 and the distal small-diameter portion 112.

The mark 53 is configured in the same manner as the mark 43 in the fourth embodiment, except that the mark 53 contains a radiopaque material. As the radiopaque material, those which are known can be used, examples of which include tungsten and barium sulfate.

The resin coating portion 52 has a first coating material 421, which is the same as that in the fourth embodiment, and a second coating material 522 coating the first coating material 421. The second coating material 522 differs from the second coating material 422 of the fourth embodiment in that it does not contain any radiopaque material, but it is the same as the second coating material 422 in all other respects.

The guide wire 50 in the fifth embodiment has the coil 51 at its distal portion 111, which makes it possible to check the position of the distal end of the guide wire under endoscopy. In addition, because the mark 53 is radiopaque, the position of a flexible portion 115 can be grasped or determined under radioscopy.

With both the coil 51 and the mark 53 being radiopaque, the size (length) of a stenosed part in the inside of a body lumen can be measured under radioscopy using the known or predetermined distance between the coil 51 and the mark 53 as a reference. The benefits associated with the fifth embodiment of the guide wire 50 are the same as those associated with the fourth embodiment.

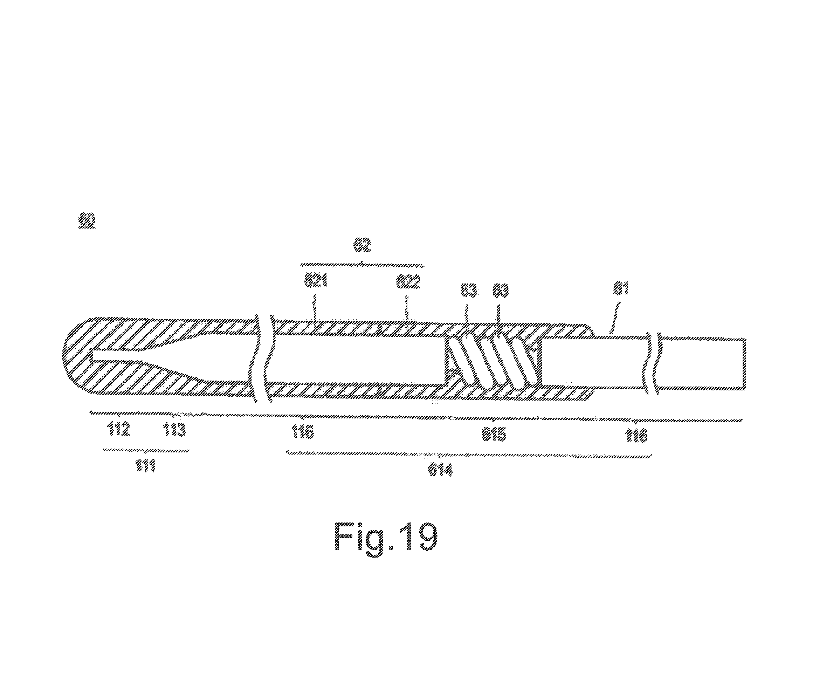

Referring to FIG. 19, a guide wire 60 according to a sixth embodiment includes a flexible wire 61 as a core material, a resin coating portion 62 with which the wire 61 is coated, a flexible portion 615 in an intermediate portion of the wire 61, and an anchor member 63 provided at a position of the resin coating portion 62 at which the flexible portion 615 is coated with the resin coating portion 62.

The wire 61 has an elongate body section 614, and a distal portion 111 decreased in outer diameter from the body section 614. The material forming the wire 61 is the same as that forming the wire 11 in the first embodiment. In addition, the configuration of the distal portion 111 is the same as in the first embodiment.

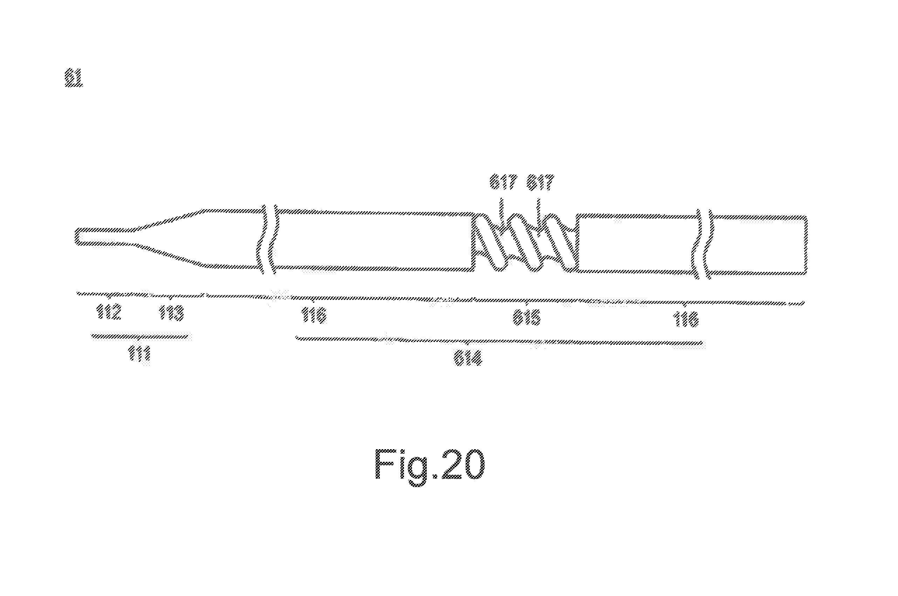

As shown in FIG. 20, the flexible portion 615 has a configuration in which a spiral groove 617 is formed in the outer peripheral surface of a part of the body section 614. The flexible portion 615 is lower in flexural rigidity than the portions 116 of the body section 614 other than the flexible portion 615.

The anchor member 63 is provided along the groove 617. A part of the anchor member 63 protrudes radially outward from the inside of the groove 617. The anchor member 63 is formed from a resin material. As the resin material forming the anchor member 63, known resin materials can be appropriately applied. Examples of the resin material for forming the anchor member 63 include polysulfone, polyimides, polyether-ether ketone, polyallylene ketone, polyphenylene sulfide, polyallylene sulfide, polyamide-imides, polyether imides, polyimide sulfone, polyallyl sulfone, polyallyl ether sulfone, polyesters, polyether sulfone, and fluororesins such as polytetrafluoroethylene (PTFE), ethylene-tetratfluoroethylene copolymer (ETFE), etc., which may be used either singly or in combination of two or more of them. Other examples than the just-mentioned include epoxy resins, phenolic resins, polyesters (unsaturated polyesters), polyimides, silicone resins, polyurethane, etc., which may be used either singly or in combination of two or more of them. The resin material forming the anchor member 63 is preferably one which exhibits good bond strength with the wire 61 and which is not easily peeled from the wire 61.

The resin material forming the anchor member 63 contains radiopaque filler. Examples of the radiopaque filler include powders of metals such as tungsten, gold, platinum, etc., and powders of metallic oxides such as barium sulfate, barium carbonate, bismuth oxide, etc.

The resin material forming the anchor member 63 contains a pigment so that the anchor member 63 differs in color from the outer surface of the wire 61. The resin coating portion 62 is light-transmitting, and the anchor member 63 is visually discernible through the resin coating portion 62. The anchor member 63 is thus configured to be a visually discernible mark.

The contrast between the anchor member 63 and the outer surface of the wire 61 is preferably high, from the viewpoint of visual discernibleness or visual differentiation. An example of the method by which the contrast can be enhanced is a setting such that the outer surface of the wire 61 is silver white (metallic color), gray or black in color, whereas the anchor member 63 is red or yellow in color. Another example of the method for successfully enhancing the contrast is a setting such that the outer surface of the wire 61 is black, charcoal gray, dark brown, dark-blue, purple or the like in color, whereas the anchor member 63 is yellow, greenish-yellow, orange or the like in color. As the pigment, those which have been known can be applied, which may be either organic or inorganic. Two or more pigments may be used in a mixed state.

The resin coating portion 62 has a proximal coating portion 622 with which a part of the body section 614 is coated, and a distal coating portion 621 with which the entire distal portion 111 is coated, The proximal coating portion 622 coats the outer circumference of the entire flexible portion 615, and the vicinity of the flexible portion 615. The distal coating portion 621 coats the distal portion 111 entirely. The distal coating portion 621 and the proximal coating portion 622 are integrally provided so that the proximal end of the distal coating portion 621 and the distal end of the proximal coating portion 622 contact each other and are fixed to each other.

The proximal coating portion 622, preferably, has compatibility with the anchor member 63. The term "compatibility" here means "an ability with which two or more substances are able to homogeneously mix with one another without causing inconvenient separation (spewing, blooming) and without causing a chemical reaction" (citation from "Eiwa Plastic Kogyo Jiten Dai 5 Han" (English-Japanese Dictionary of Plastic Industry, 5th Ed.), published on May 25, 1992 by Kogyo Chosakai Publishing Co., Ltd., p. 187).

The proximal coating portion 622 and the anchor member 63 can have compatibility with each other by containing the same resin, or can have compatibility with each other by containing resin materials having the same group. For instance, the proximal coating portion 622 and the anchor member 63 can be compatible with each other by containing respective resin materials having an "imide group," such as polyamide-imide and polyimide, polyether-imide and polyimide, polyamide-imide and polyether-imide, or by containing respective resin materials having a "sulfone group," such as polysulfone and polyether-sulfone.

Where the proximal coating portion 622 contains a fluororesin such as polytetrafluoroethylene (PTFE), ethylene-tetrafluoroethylene copolymer (ETFE), etc, it is possible to contrive a reduction in the friction on the outer surface thereof.

The distal coating portion 621 may be formed from the same resin material as that for the proximal coating portion 622. The distal coating portion 621 is radiopaque, and contains radiopaque filler such as the above-mentioned metallic powders and metallic oxide powders.

The distal coating portion 621 preferably has compatibility with the proximal coating portion 622. Like in the case where the proximal coating portion 622 and the anchor member 63 have compatibility with each other, the distal coating portion 621 has compatibility with the proximal coating portion 622, for example, by containing the same resin material as that forming the proximal coating portion 622, or by containing a resin material which has the same group as that possessed by the resin material forming the proximal coating portion 622.

The method for operating the guide wire 60 in this embodiment as above-described and the endoscope E are the same as those in the first embodiment.

The operation and effect of the sixth embodiment will be described below.

The guide wire 60 is liable to be bent at its flexible portion 615. Therefore, even in the case where the distal end of the endoscope E is held in a position looking up at the duodenal papilla P, the disposition of the flexible portion 615 between the distal end of the endoscope E and the duodenal papilla P as shown in FIG 12 helps ensure that in the condition where the guide wire portion on the distal side of the flexible portion 615 is inserted in the duodenal papilla P, a portion of the guide wire on the proximal side opposite to the distal side can be bent so as to make contact with the corner portion E3 and the distal end E4 of the treatment instrument elevator base E2. Consequently, the function of fixing the guide wire 60 onto the endoscope E can be relatively easily displayed independently of the positional relationship between the distal end of the endoscope E and the duodenal papilla P.

For ease in understanding the operation and effect of the guide wire, the guide wire is represented only by a black line in FIG. 12. As above-mentioned, however, the anchor member 63 is different in color from the outer surface of the wire 61 and is visually discernible through the proximal coating portion 622. Therefore, the operator can relatively easily grasp the position of the anchor member 63 and, hence, the position of the flexible portion 615 on the basis of the function of the image pick-up section E52 provided at the distal end of the endoscope E.

Because the anchor member 63 is radiopaque and the radiopacity can be obtained at the body section 614 of the guide wire 60, it is possible to grasp or determine the position of the body section 614, particularly the position of the flexible portion 615, even in the case where, for example, the anchor member 63 is so located as not to be visually detectable through the function of the image pick-up section E52.

By virtue of the distal coating portion 621 being radiopaque and the distance between the distal coating portion 621 and the anchor member 63 being a fixed known distance, the length of a predetermied part in a body lumen such as a stenosed part can be measured under radioscopy.

As mentioned, the distal coating portion 621 is provided integrally with the proximal coating portion 622 and the proximal coating portion 622 ties the distal coating portion 621 securely, and so the distal coating portion 621 can be inhibited or prevented from exfoliation or slipping-off.

The distal portion 111 is decreased in outer diameter from the body section 614 and the flexibility of the wire 61 increases along the distal direction. Therefore, the guide wire 60 is excellent in operationality and safety.

In the guide wire 60, the anchor member 63 is provided along the groove 617, whereby the anchor member 63 and the wire 61 are inhibited or prevented from being easily disengaged from each other. The anchor member 63 is formed from a resin material, like the proximal coating portion 622, so that the resin coating portion 62 is in better connection with the anchor member 63, as compared with the case where the anchor member 63 is formed from a metallic material, for example. Therefore, the resin coating portion 62 is secured firmly to the wire 61 through the anchor member 63, and is not readily susceptible to exfoliation.

Because the anchor member 63 is formed from a resin material, it is more inexpensive as compared with the case of being formed from a metallic material. And because the anchor member 63 is formed from a resin material, the wire 61 is more flexible and the guide wire 60 is better in operationality and safety, as compared with the case where the anchor member 63 is formed from a metallic material.

The spiral form of the groove 617 makes the wire 61 less susceptible to plastically deforming when bent, as compared with the case where a plurality of mutually separate annular grooves are formed around the wire 61, for example.

As mentioned above, the anchor member 63 and the proximal coating portion 622 are compatible with each other, and so a stronger connection is achieved between the resin coating portion 62 and the anchor member 63. Consequently, the resin coating portion 62 is more effectively inhibited or prevented from peeling or slipping-off.

The anchor member 63 protrudes radially outward from the groove 617 and the area of contact between the proximal coating portion 622 and the anchor member 63 is relatively large. The resin coating portion 62 is thus more effectively inhibited or prevented from exfoliation or slipping-off.

Because the anchor member 63 is formed from a resin material containing radiopaque filler and is therefore radiopaque, the position of the guide wire 60 can be grasped under radioscopy.

The present invention is not limited to the above-mentioned embodiments, and various modifications are possible within the scope of the claims. For instance, the flexible portion is not restricted to the above-mentioned ones, and includes any other form that offers a local reduction in flexural rigidity. For example, the body section may be partially smashed by press working so as to form a flexible portion which is elliptic in cross-section. In this case, preferably two flexible portions having respective elliptic cross-sectional shapes with the major-axis directions intersecting each other are formed adjacently, in order that the guide wire can be easily bent in different directions at the flexible portions.

While the guide wire is inserted into the duodenal papilla through the catheter C1 in the above embodiments, the guide wire may be inserted into the duodenal papilla directly from the endoscope, without use of the catheter C1.

The detailed description above describes features and aspects of embodiments of a guide wire for endoscopes and operational aspects associated with the use of the guide wire. The invention is not limited, however, to the precise embodiments and variations described. Various changes, modifications and equivalents could be effected by one skilled in the art without departing from the spirit and scope of the invention as defined in the appended claims. It is expressly intended that all such changes, modifications and equivalents which fall within the scope of the claims are embraced by the claims.

## Claims

1. A guide wire comprising:
a wire having a body section and a distal portion, the distal portion of the wire being distal of the body section and possessing a distal-most end, the distal portion decreasing in outer diameter toward the distal-most end of the distal portion;
a resin coating portion covering at least a part of an axial extent of the body section and covering the distal portion, the resin coating portion being an exposed outer surface of the guide wire, the exposed outer surface being a smooth outer surface, the resin coating portion comprising a resin;
a visually discernible mark at the resin coating portion, the visually discernable mark at the resin coating portion being visually distinguishable from areas of the resin coating portion on opposite axial sides of the visually discernible mark; and
the body section comprising a flexible portion which is a part of the body section, the flexible portion being lower in flexural rigidity than other portions of the body section positioned on both axial ends of the flexible portion, and the visually discernible mark being provided at a position of the resin coating portion which overlies the flexible portion so that the visually discernible mark axially overlaps the flexible portion.

2. The guide wire according to Claim 1, wherein the visually discernible mark includes a material having a radiopaque property.

3. The guide wire according to Claim 1, wherein the body section includes a plurality of axially spaced apart flexible portions with intervals between the axially spaced apart flexible portions, each of the flexible portions comprising a reduced outer diameter portion of the wire which possesses a reduced outer diameter relative to the intervals, each reduced outer diameter portion having the reduced outer diameter around an entire circumferential extent of the wire.

4. The guide wire according to Claim 1, wherein the flexible portion is covered by the resin coating portion which is a first resin coating portion, and a proximal-side resin coating portion covering a part of the wire positioned proximally of the first resin coating portion, the proximal-side resin coating portion being composed of resin different from the resin comprising the first resin coating portion.

5. The guide wire according to Claim 1, wherein the flexible portion is neck-shaped such that the neck-shaped portion possesses a reduced outer diameter relative to other portions of the wire adjoining the flexible portion; the reduced outer diameter extending around an entire circumference of the wire.

6. The guide wire according to Claim 1, wherein the flexible portion has a spiral-shaped groove in an outer peripheral surface of the body section.

7. The guide wire according to Claim 1, wherein the flexible portion includes a center relative to a longitudinal extent of the body section, the center of the flexible portion being spaced proximally from a distal-most end of the resin coating portion by not less than 150 mm.

8. A medical instrument comprising:
an endoscope with a treatment instrument insertion channel and a treatment instrument elevator base at a distal end portion of the treatment instrument insertion channel, the treatment instrument insertion channel possessing a proximal opening and a distal opening;
a guide wire insertable into the treatment instrument insertion channel by way of the proximal opening so that the guide wire extends along the treatment instrument insertion channel and a distal end portion of the guide wire extends distally beyond the distal opening of the treatment instrument insertion channel to be inserted into a duodenal papilla, the guide wire possessing an exposed outer surface;
the guide wire including a wire comprised of an elongated body section and a distal portion located distal of the body section and possessing an outer diameter decreasing in a distal direction of the distal portion, the guide wire also comprising a resin coating portion covering at least a part of an axial extent of the body section and the distal portion, the resin coating possessing an outer surface forming the exposed outer surface of the guide wire, the outer surface of the resin coating being smooth,
the guide wire having a flexible portion lower in flexural rigidity than other portions of the guide wire, the flexible portion being disposed along a length of the guide wire such that with the guide wire extending along the treatment instrument insertion channel and the distal end portion of the guide wire positioned in the duodenal papilla the flexible portion is distal of the opening.

9. The medical instrument according to Claim 8, wherein the resin coating includes a visually discernible mark that is visually distinguishable from areas of the resin coating portion on immediately opposite axial sides of the visually discernible mark, and the visually discernible mark axially overlapping the flexible portion.

10. The medical instrument according to Claim 9, wherein the body section includes a plurality of axially spaced apart flexible portions each possessing a flexural rigidity lower than portions of the guide wire on opposite sides of each flexible portion, the resin coating including a plurality of axially spaced apart visually discernible marks each of which is visually distinguishable from areas of the resin coating portion on immediately opposite axial sides of the visually discernible mark, and each visually discernible mark axially overlapping one of the flexible portions.

11. The medical instrument according to Claim 8, wherein the flexible portion is covered by the resin coating portion which is a first resin coating portion, and a proximal-side resin coating portion covering a part of the wire positioned proximally of the first resin coating portion, the proximal-side resin coating portion being composed of resin different from the resin comprising the first resin coating portion.

12. The medical instrument according to Claim 8, wherein the flexible portion includes a spiral-shaped groove in an outer peripheral surface of the body section, the resin coating covering the spiral-shaped groove and axially overlapping the spiral-shaped groove.

13. The medical instrument according to Claim 8, wherein the flexible portion includes a center relative to a longitudinal extent of the body section, the center of the flexible portion being spaced proximally from a distal-most end of the resin coating portion by not less than 150 mm.
